(19) Europäisches Patentamt
European Patent Office
Office européen des brevets

(11) **EP 4 007 528 B1**

(12) **FASCICULE DE BREVET EUROPEEN**

(45) Date de publication et mention
de la délivrance du brevet:
**18.12.2024   Bulletin 2024/51**

(21) Numéro de dépôt: **20756933.6**

(22) Date de dépôt: **29.07.2020**

(51) Classification Internationale des Brevets (IPC):
***A61B 8/08*** *(2006.01)*       ***A61B 8/00*** *(2006.01)*

(52) Classification Coopérative des Brevets (CPC):
**A61B 8/0883; A61B 8/485; A61B 8/543;**
A61B 8/5223

(86) Numéro de dépôt international:
**PCT/FR2020/051396**

(87) Numéro de publication internationale:
**WO 2021/019186 (04.02.2021 Gazette 2021/05)**

(54) **DISPOSITIF CARDIAQUE**

KARDIALE VORRICHTUNG

CARDIAC DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **01.08.2019   FR 1908859**

(43) Date de publication de la demande:
**08.06.2022   Bulletin 2022/23**

(73) Titulaires:
• **Institut National de Recherche en
Informatique et en Automatique (INRIA)
78153 Le Chesnay Cedex (FR)**
• **INSTITUT NATIONAL DE LA SANTE ET DE LA
RECHERCHE
MEDICALE
75654 Paris Cedex 13 (FR)**

(72) Inventeurs:
• **CHAPELLE, Dominique
78153 Le Chesnay Cedex (FR)**
• **MOIREAU, Philippe
78153 Le Chesnay Cedex (FR)**
• **PERNOT, Mathieu
75013 Paris Cedex 13 (FR)**
• **TANTER, Mickaël
75013 Paris Cedex 13 (FR)**

(74) Mandataire: **Vidon Brevets & Stratégie
16B, rue de Jouanet
BP 90333
35703 Rennes Cedex 7 (FR)**

(56) Documents cités:
**US-A1- 2018 064 412**

• VILLEMAIN OLIVIER ET AL: "Myocardial
Stiffness Evaluation Using Noninvasive Shear
Wave Imaging in Healthy and Hypertrophic
Cardiomyopathic Adults", JACC:
CARDIOVASCULAR IMAGING, ELSEVIER,
AMSTERDAM, NL, vol. 12, no. 7, 2 July 2019
(2019-07-02), pages 1135 - 1145, XP085727206,
ISSN: 1936-878X, [retrieved on 20180314], DOI:
10.1016/J.JCMG.2018.02.002
• OLIVIER VILLEMAIN: "Myocardial Stiffness
Evaluation Using Noninvasive Shear Wave
Imaging in Healthy and Hypertrophic
Cardiomyopathic Adults - APPENDIX", JACC:
CARDIOVASCULAR IMAGING, vol. 12, no. 7, 2
July 2019 (2019-07-02), XP055705189
• CORREIA M ET AL: "3D elastic tensor imaging in
weakly transversely isotropic soft tissues",
PHYSICS IN MEDICINE AND BIOLOGY,
INSTITUTE OF PHYSICS PUBLISHING, BRISTOL
GB, vol. 63, no. 15, 25 July 2018 (2018-07-25),
pages 155005, XP020328996, ISSN: 0031-9155,
[retrieved on 20180725], DOI:
10.1088/1361-6560/AACFAF

**Description**

La présente invention concerne le domaine de la mesure cardiaque, et plus particulièrement de la mesure cardiaque non-invasive. La mesure cardiaque recouvre les mesures de grandeurs physiques du coeur d'un patient, en particulier de paramètres mécaniques.

Les récents progrès en imagerie, en particulier par ultrason ou par résonnance magnétique, ont permis d'accéder à des grandeurs jusqu'ici mesurées uniquement par des méthodes in situ. De telles méthodes in situ impliquent un accès au coeur par opération chirurgicale ou par cathétérisation. De fait, ces méthodes sont invasives et contraignantes. De plus, elles ne peuvent être réalisées qu'au sein d'un établissement adapté, avec des réglementations strictes.

A contrario, les mesures non-invasives sont plus aisées à mettre en oeuvre. Cependant, ces mesures ne permettent pas de mesurer toutes les grandeurs utiles ou facilement exploitables. On connaît ainsi les mesures de la pression artérielle radiale par des capteurs près de la peau d'un patient, par exemple via un bracelet, un capteur au doigt, ou une montre connectée. Cependant, le lien entre la pression artérielle radiale et des grandeurs physiques au sein du coeur, par exemple la pression artérielle centrale, est imprécis et indirect.

On connaît également la mesure par élastographie cardiaque, via résonance magnétique ou ultrasons. Cette technique est non-invasive et permet de mesurer la raideur apparente du tissu cardiaque. La raideur apparente est une grandeur qui varie au cours d'un battement cardiaque et selon l'endroit mesuré. Cette raideur apparente pourrait permettre en théorie de remonter à d'autres grandeurs cardiaques utiles, telles que les modules d'élasticité, la pression cardiaque, la contrainte tissulaire, etc. Cependant, il n'existe pas de relation directe entre cette mesure et ces grandeurs cardiaques. Des corrélations ont déjà été observées entre la raideur apparente et la pression cardiaque, mais elles présentent une très forte variabilité d'un individu à l'autre.

Dans la publication "Myocardial Stiffness Evaluation Using Noninvasive Shear Wave Imaging in Healthy and Cardiomyopathie Adults, O. Villemain et.al, JACC: Cardiovascular Imaging, Vol. 12, No. 7", il est proposé d'utiliser l'imagerie par ondes de cisaillement pour analyser la rigidité myocardique diastolique.

Le document WO2016156989 divulgue également la possibilité d'utiliser des ondes de cisaillement pour l'imagerie cardiaque.

L'invention vient améliorer la situation. À cet effet l'invention propose un dispositif cardiaque, comprenant un émetteur agencé pour émettre au moins une onde, une sonde agencée pour mesurer une onde de cisaillement suscitée par une onde issue de l'émetteur, un détecteur agencé pour détecter une phase de systole dans un signal électrocardiographique, et un estimateur agencé pour déterminer, pendant au moins un cycle cardiaque, la vitesse de propagation d'une pluralité d'ondes de cisaillement suscitées par l'émission d'ondes selon plusieurs directions vers le coeur d'un patient, pour déterminer à l'aide du détecteur celle qui présente durant la phase de systole une vitesse de propagation maximale, et pour en tirer une contrainte active cardiaque. Ce dispositif est avantageux, car il permet d'accéder directement à la contrainte active dans le tissu cardiaque. La contrainte active est une force par unité de surface. Cette contrainte active permet un accès direct et non-invasif à une multitude de grandeurs cardiaques utiles. On peut par exemple tirer de la contrainte active la pression intra-ventriculaire. On peut également détecter des pathologies cardiaques à partir d'une mesure de contrainte active, et par exemple observer les séquelles d'un infarctus.

Dans diverses variantes, le dispositif peut présenter une ou plusieurs des caractéristiques suivantes :

- le dispositif cardiaque comprend un calculateur agencé pour déterminer une pression ventriculaire à partir d'une épaisseur de paroi ventriculaire, d'un rayon de cavité ventriculaire et de la contrainte active déterminée par l'estimateur,
- le dispositif cardiaque comprend un analyseur d'image agencé pour déterminer l'épaisseur de paroi ventriculaire et le rayon de cavité ventriculaire sur la base d'au moins une image cardiaque,
- l'au moins une image cardiaque est tirée d'une imagerie par ondes de cisaillement,
- le dispositif cardiaque comprend en outre un imageur agencé pour fournir l'imagerie par ondes de cisaillement à partir d'au moins une onde de cisaillement mesurée par la sonde,
- l'au moins une onde issue de l'émetteur est un ultrason de compression,
- l'émetteur et la sonde sont mis en oeuvre par une sonde d'échocardiographie bidimensionnelle qui présente une direction de sonde définissant une direction de propagation autour de laquelle elle est pivotable, et dans lequel l'estimateur est agencé pour déterminer la vitesse de propagation maximale sur la base de mesures réalisées par la sonde pour trois directions coplanaires et non colinéaires,
- l'émetteur et la sonde sont mis en oeuvre par une sonde ultrason tridimensionnelle,
- la sonde ultrason tridimensionnelle est une sonde d'échocardiographie tridimensionnelle.

D'autres caractéristiques et avantages de l'invention seront exposés en détail dans la description ci-après, faite en référence aux dessins annexés, sur lesquels :

- [Fig. 1] représente une vue schématique du dispositif cardiaque selon l'invention,
- [Fig. 2] représente une vue schématique du dispositif cardiaque de la figure 1, dans une configuration de mesure de pression ventriculaire, et
- [Fig. 3] représente une vue schématique d'une autre configuration du dispositif cardiaque de la figure 2.

Les dessins annexés contiennent, pour l'essentiel, des éléments de caractère certain. Ils pourront donc non seulement servir à mieux faire comprendre la présente invention, mais aussi contribuer à sa définition, le cas échéant.

La description est suivie d'une Annexe A comprenant des formules mathématiques. Cette annexe fait partie intégrante de la description.

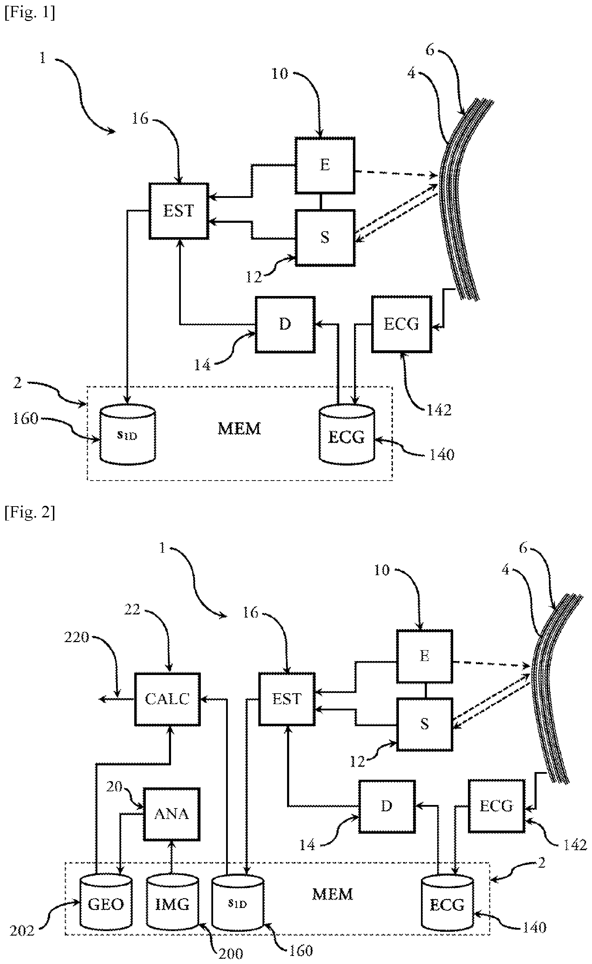

Il est maintenant fait référence à la figure 1.

Un dispositif cardiaque 1 selon l'invention comprend une mémoire 2, un émetteur 10 et une sonde 12.

La mémoire 2 peut être n'importe quel type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. Les données stockées dans la mémoire 2 peuvent être effacées après que le dispositif cardiaque 1 a effectué ses tâches, ou conservées. Les données calculées par le dispositif cardiaque peuvent être stockées sur la mémoire 2 ou sur tout type de mémoire similaire à celle-ci.

Des techniques d'observation non invasives de milieux anisotropes (par exemple fibreux) par ultrasons ont été développées. Par exemple, la demande PCT/FR2015/050058 porte sur la mesure de caractéristiques mécaniques au sein de milieux anisotropes, par observation de la propagation d'ultrasons au sein de celui-ci.

L'émetteur 10 est ici un dispositif apte à émettre des ultrasons. Un ultrason émis par l'émetteur 10 dirigé vers un tissu fibreux 4, par exemple la paroi d'un coeur 6, suscite en réaction à son entrée dans le tissu fibreux une onde de cisaillement se propageant dans ce tissu fibreux dans un plan orthogonal à la direction d'émission de l'ultrason. Cette propagation est observée par la sonde 12 via des ultrasons de compression émis à haute cadence, comme décrit dans la demande PCT/FR2015/050058.

Le dispositif cardiaque 1 comprend également un détecteur 14 et un estimateur 16. Le détecteur 14 détermine les moments où le coeur observé est en phase de systole, c'est-à-dire lorsque le coeur se contracte. L'estimateur 16 est relié à l'émetteur 10, à la sonde 12 et au détecteur 14 de sorte à pouvoir recevoir les données qui en proviennent. L'estimateur 16 peut tirer de ces données une ou des grandeurs physiques, en particulier une contrainte active 160 du tissu cardiaque.

Afin de réaliser une mesure de propagation, l'émetteur 10 et la sonde 12 sont mis en contact de la peau d'un patient dont on souhaite observer le coeur 6, et dirigés vers ce dernier. L'émetteur 10 et la sonde 12 fonctionnent de concert pour observer la propagation d'ondes de cisaillement au sein de tissus cardiaques.

L'émetteur 10 génère un faisceau d'ultrasons focalisés durant une courte durée, dans une direction d'émission vers le coeur 6. Ce faisceau provoque, lorsqu'il atteint le coeur, le déplacement du tissu cardiaque 4 par effet de pression de radiation acoustique. Ce déplacement prend la forme d'une onde de cisaillement qui se propage au sein du tissu cardiaque 4 dans une direction de propagation sensiblement orthogonale à la direction d'émission.

La sonde 12 observe la propagation de l'onde de cisaillement. Pour ce faire, la sonde 12 émet à haute cadence des ultrasons sous forme d'ondes de compression en direction du coeur 6. Ces ultrasons sont réverbérés par le tissu cardiaque 4 se déformant sous l'effet de l'onde de cisaillement. Les ondes réverbérées sont captées par la sonde 12, ce qui permet d'observer le déplacement du tissu cardiaque 4. La cadence à laquelle la sonde 12 émet des ultrasons est supérieure à 300Hz, voire à 500Hz. Chaque mesure de propagation d'onde de cisaillement prise individuellement est réalisable en environ quelques millisecondes. La sonde 12 peut ainsi observer avec une grande précision et plusieurs centaines de fois par seconde la propagation de l'onde de cisaillement au sein du tissu cardiaque 4. La sonde 12 détermine en outre la vitesse de propagation de l'onde de cisaillement. La sonde 12 peut en outre associer la vitesse de propagation déterminée de l'onde de cisaillement à sa direction de propagation.

Afin de travailler de concert, l'émetteur 10 et la sonde 12 peuvent être commandés par un utilisateur et/ou par un contrôleur. Dans l'exemple décrit ici, l'émetteur 10 et la sonde 12 sont reliés l'un à l'autre pour communiquer ensemble.

La Demanderesse a remarqué que la vitesse de propagation d'une onde de cisaillement au sein d'un tissu fibreux cardiaque est maximale lorsque cette onde se propage selon une direction tangente à la direction des fibres du tissu. Ainsi, en réalisant plusieurs mesures de vitesse de propagation le long de différentes directions de propagation, il est possible de déterminer la direction des fibres du tissu fibreux, et la vitesse de propagation d'une onde de cisaillement le long de ces fibres.

En effet, le problème de la détermination de la propagation de l'onde de cisaillement le long des fibres peut être résumé sous la forme de l'équation (1) de l'Annexe A, qui est également connue sous le nom d'équation de Christoffel. Dans cette équation, le premier terme est également connu en tant que matrice de Christoffel M, r est la masse volumique du tissu cardiaque, F est le vecteur de polarisation de l'onde, et V est la vitesse de propagation de l'onde plane.

Lors de ses travaux, la Demanderesse a découvert que, lors de la phase de systole, le terme de tension active domine dans le tenseur de contraintes du milieu fibreux cardiaque. Cela lui a permis de reformuler la matrice de Christoffel M de l'équation (1) sous la forme générale de l'équation (2) de l'Annexe A, où $n_1$ est le cosinus de l'angle entre la direction de propagation de l'onde et la direction de fibre, et $s_{1D}$ est la contrainte active. Dans cette équation, la matrice M est exprimée dans un repère dont le premier vecteur est la direction de fibre. En s'intéressant aux termes de polarisations transverses à la fibre dans le tenseur (deuxième et troisième terme de la diagonale de la matrice M), qui valent $s_{1D*}n_1^2$, et en les réinjectant dans l'équation (1) de l'Annexe A, la Demanderesse a alors établi l'équation (3) de l'Annexe A. La Demanderesse a ensuite établi la relation entre vitesse maximale de propagation, masse volumique du tissu cardiaque et contrainte active, exprimée sous la forme de l'équation (4) en Annexe A, qui correspond au cas où ni est égal à 1.

La masse volumique r vaut ici sensiblement 1060 kg/m$^3$. La masse volumique r présente une faible variabilité entre individus, car la proportion d'eau au sein du tissu cardiaque y est élevée. De ce fait, les travaux de la Demanderesse peuvent donc être utilisés pour la mesure de la contrainte active chez l'être humain.

Pour déterminer la contrainte active 160, il est nécessaire de repérer d'abord la phase de systole. À cet effet, le détecteur 14 détermine une phase de systole sur la base d'un signal électrocardiographique 140. Le signal électrocardiographique 140 est ici issu d'un électrocardiographe 142 intégré au dispositif cardiaque 1. En variante, le signal électrocardiographique 140 peut provenir d'un appareil extérieur au dispositif cardiaque 1 et être stocké dans la mémoire 2. Cette variante n'est pas représentée sur les figures.

La phase de systole dure entre 200 et 400 millisecondes. Compte tenu de la durée de la mesure réalisable par l'émetteur 10 et la sonde 12, de l'ordre de quelques millisecondes, il est ainsi possible de réaliser un grand nombre de mesures de vitesse de propagation durant une même phase de systole.

L'estimateur 16 est ici un programme exécuté par le processeur d'un ordinateur. En variante, il pourrait être mis en oeuvre de manière différente au moyen d'un processeur dédié. Par processeur, il doit être compris tout processeur adapté aux traitements de données décrits dans la présente demande. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeurs et de circuits électroniques peut également être envisagée.

L'estimateur 16 reçoit un jeu de vitesses de propagation déterminées par la sonde 12, chaque vitesse de propagation étant associée à la direction de propagation de son onde de cisaillement. L'estimateur 16 reçoit l'information issue du détecteur 14 indiquant la phase de systole. Sur la base du jeu de vitesses de propagation, l'estimateur 16 détermine la vitesse de propagation maximale de l'onde de cisaillement en phase de systole.

Pour ce faire, un utilisateur ou un contrôleur externe commande la direction d'émission des ondes émises par l'émetteur 10, de sorte qu'une pluralité de mesures de vitesse de propagation peut être réalisée. À partir de celles-ci, l'estimateur 16 détermine la vitesse de propagation maximale de l'onde de cisaillement au sein du tissu cardiaque 4, c'est-à-dire la direction des fibres du tissu cardiaque 4.

Pour cela, l'estimateur 16 peut réaliser une pluralité de mesures de vitesse de propagation selon une pluralité de directions de propagation réparties dans le plan tangent à la paroi cardiaque, et prendre la plus grande des vitesses de propagation mesurées. Cette pluralité de directions de propagation peut par exemple être choisie de sorte que leur distribution angulaire soit sensiblement régulière. L'estimateur 16 peut en outre exploiter la formule (3) pour, à partir d'au moins trois mesures de vitesse de propagation tirées de directions de propagation des ondes émises par l'émetteur 10 qui sont coplanaires et non colinéaires, interpoler une sinusoïde et en tirer la vitesse de propagation maximale de l'onde de cisaillement au sein du tissu cardiaque 4.

Après avoir déterminé la vitesse de propagation maximale, l'estimateur 16 en tire la contrainte active 160, selon la formule (4) de l'Annexe A.

Dans un premier mode de réalisation de l'invention, l'émetteur 10 et la sonde 12 sont mis en oeuvre dans une sonde d'échocardiographie bidimensionnelle. Les ultrasons sont émis par l'émetteur 10 et focalisés dans le tissu cardiaque. La sonde 12 émet des ondes non focalisées à très haute cadence pour imager la propagation de l'onde cisaillement dans un plan. En pivotant ce plan sensiblement autour de cet axe de sonde principal, les ultrasons balaient un cône ou une portion de cône autour de cet axe de sonde principal. Un utilisateur peut ainsi tourner manuellement la sonde d'échocardiographie bidimensionnelle pour assurer une pluralité de mesures de vitesse de propagation selon au moins trois directions de propagation. La mesure des directions de propagation peut être manuelle, automatisée ou guidée. Ainsi, en quelques secondes et par une manipulation simple, il est possible de déterminer la vitesse de propagation maximale des ondes de cisaillement comme décrit ci-dessus. Le fait de pouvoir mettre en oeuvre émetteur 10 et sonde 12 dans une sonde d'échocardiographie bidimensionnelle est très avantageux, car ces sondes sont peu coûteuses et présentes dans la plupart des établissements médicaux. Plus généralement, dans ce mode de réalisation, le dispositif cardiaque 1 tout entier peut être mis en oeuvre dans un dispositif d'échocardiographie bidimensionnel classique.

Dans un deuxième mode de réalisation, l'émetteur 10 et la sonde 12 sont mis en oeuvre au sein d'une sonde d'échocardiographie tridimensionnelle. Dans cette configuration, l'émetteur 10 émet des ultrasons dans une pluralité de directions de propagation balayant un angle solide non nul, par exemple un cône autour d'un axe de sonde principal. La mesure de la vitesse de propagation maximale est ici assurée sans avoir besoin de déplacer ou de pivoter la sonde d'échocardiographie tridimensionnelle. La détermination de la contrainte active est ainsi plus fiable, rapide et précise. En outre, le dispositif cardiaque 1 présente ici l'avantage de pouvoir être mis en oeuvre dans un dispositif d'échocardiographie tridimensionnel classique.

Dans un troisième mode de réalisation, le dispositif cardiaque 1 est mis en oeuvre dans un appareil autonome du type décrit dans la demande PCT/FR2015/050058, différent d'un dispositif d'échocardiographie. Ce dispositif spécialisé permet d'observer des mesures de propagation quasiment équivalentes à celles que permet une sonde d'échocardiographie tridimensionnelle, tout en étant plus simple de conception et moins coûteux. L'émetteur 10 et la sonde 12 sont agencés pour réaliser la mesure de vitesse de propagation maximale sans avoir besoin de déplacer ou de pivoter l'appareil autonome. La portion de l'appareil autonome contenant l'émetteur 10 et la sonde 12 peut être d'une taille inférieure à une distance intercostale. Cette petite taille augmente la précision des mesures par la sonde 12. La mise en oeuvre du dispositif cardiaque 1 dans un appareil autonome de ce type permet d'en simplifier la fabrication. En outre, dans cette configuration, le dispositif cardiaque 1 peut être interfacé avec un appareil médical classique distinct, auquel il peut communiquer des données, en particulier une contrainte active 160 déterminée.

Il est maintenant fait référence à la figure 2.

La Demanderesse a découvert un moyen de déterminer une pression ventriculaire à partir de la contrainte active cardiaque. Pour ce faire, la Demanderesse a modélisé le comportement mécanique en équilibre statique d'une cavité ventriculaire sensiblement sphérique d'épaisseur d et de rayon R. Sous une pression p, avec e = d/R, la Demanderesse a établi la formule (5) en annexe A. Cette formule (5) relie la contrainte active 160, l'épaisseur d et le rayon R à la pression interne 220, où $a_{cor}$ est un facteur de correction. L'épaisseur d et le rayon R sont des données géométriques de la cavité ventriculaire.

Dans le mode de réalisation décrit ici, le dispositif cardiaque 1 comprend un calculateur 22. Le calculateur 22 reçoit la contrainte active 160 issue de l'estimateur 16 et des données géométriques 202. Le calculateur 22 détermine une pression ventriculaire au moyen de la formule (5) de l'Annexe A, sur la base de la contrainte active 160 et des données géométriques 202.

Le dispositif cardiaque 1 peut comprendre facultativement un analyseur d'image 20 pour générer les données géométriques 202.

L'analyseur d'image 20 récupère des données d'image 200 de la cavité ventriculaire du coeur observé. L'analyseur d'image 20 en tire les données géométriques 202 de la cavité ventriculaire. L'analyseur d'image 20 détermine au moins une épaisseur de paroi ventriculaire d et un rayon de cavité ventriculaire R de la cavité ventriculaire.

Les données d'images 200 peuvent être issues d'un procédé d'imagerie cardiaque par ultrason (échocardiographie), par résonnance magnétique (IRM), par tomodensitométrie (ou scanographie), par radioactivité (médecine nucléaire) ou par tout autre méthode d'imagerie cardiaque non invasive. Les données d'image peuvent être stockées dans la mémoire 2. Dans l'exemple décrit ici, les données d'image 200 proviennent d'un appareil tiers.

Les données géométriques 202, et les données d'image 200 le cas échéant, sont stockées en mémoire 2.

Dans le premier (respectivement deuxième) mode de réalisation l'analyseur d'image 20 est relié à la sonde d'échocardiographie bidimensionnelle (respectivement tridimensionnelle), et cette dernière fournit les données d'images nécessaires à l'analyseur d'image 20. Dans le troisième mode de réalisation, le dispositif cardiaque 1 fournit également les données d'image nécessaires à l'analyseur d'image 20. L'analyseur d'image 20 peut analyser les données d'image avec une méthode d'apprentissage automatique ou par un algorithme. L'analyseur d'image 20 peut par exemple comprendre un réseau neuronal, en particulier convolutif, afin de reconnaître une forme au sein des données d'images, de laquelle il peut tirer les données géométriques.

La mesure de l'épaisseur de paroi ventriculaire d et du rayon de cavité ventriculaire R peut être réalisée à partir d'une image échocardiographique du coeur en déterminant le contour des parois internes (endocarde) et externes (épicarde) du coeur. La segmentation peut ici être manuelle ou automatique.

En variante, on peut obtenir une image issue d'une sonde d'échocardiographie en mode temps-mouvement (TM) représentant une ligne échographique en fonction du temps. On tire de cette image une visualisation des parois cardiaques, que l'on peut ensuite segmenter manuellement ou automatiquement, pour calculer l'épaisseur d de paroi ventriculaire et le rayon de cavité ventriculaire R.

Compte tenu de l'approximation sphérique de la cavité ventriculaire, la formule (5) comprend un facteur de correction $a_{cor}$. Ce facteur de correction peut être déterminé au préalable, par exemple par simulation numérique, ou mesuré pour un groupe de patients. De manière alternative, ce facteur de correction peut être déterminé à la volée par un composant du dispositif cardiaque 1 capable de faire de l'analyse de formes géométriques ou de l'analyse d'image. Cette analyse par exemple pourrait être menée par l'analyseur d'image 20, à partir des données d'image 200. Dans l'exemple décrit ici, le facteur de correction peut varier entre 0,6 et 1, de manière préférée entre 0,75 et 0,95, et de manière encore préférée il peut être fixé à 0,9. La Demanderesse a estimé la variabilité du facteur de correction entre individus à environ 10%. Cette variabilité est bien moindre que celle des mesures de pression non invasives connues. L'utilisation du calculateur 22 et de l'analyseur d'image 20 permet ainsi d'obtenir une mesure non invasive de la pression ventriculaire 220 nettement plus précise que les méthodes classiques.

La mesure de la pression ventriculaire p de manière non invasive par le dispositif cardiaque 1 permet de diagnostiquer de multiples pathologies avec du matériel peu coûteux par un acte médical simple. On pourrait par exemple détecter une insuffisance cardiaque par la mesure de la pression ventriculaire avec un dispositif cardiaque 1 mis en oeuvre dans une sonde d'échocardiographie bidimensionnelle. Il est également possible d'utiliser cette mesure de pression non invasive dans le domaine de la resynchronisation cardiaque (« cardiac resynchronisation therapy »).

L'analyseur d'image 20 et le calculateur 22 sont des programmes exécutés par un ou plusieurs processeurs d'un ordinateur. En variante, ils pourraient être mis en oeuvre de manière différente au moyen de processeurs dédiés, ou sur des machines distinctes.

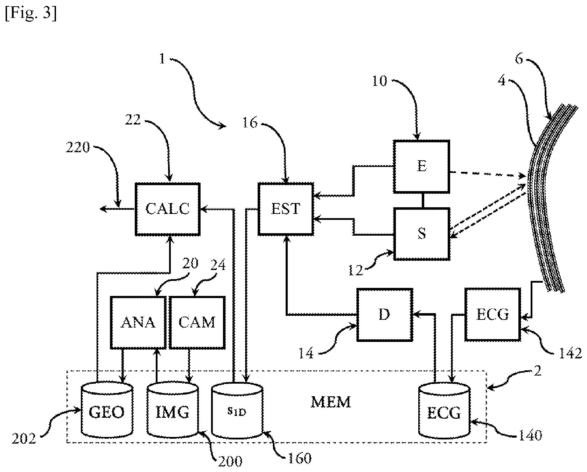

Il est maintenant fait référence à la figure 3.

Dans ce mode de réalisation, le dispositif cardiaque 1 comprend un imageur 24 pour générer les données d'image. Lorsque le dispositif cardiaque 1 est mis en oeuvre dans un dispositif d'échocardiographie, l'imageur 24 comprend les parties du dispositif d'échocardiographie réalisant une ou des échocardiographies.

Il a été décrit l'utilisation de la contrainte active pour mesurer une pression ventriculaire. Il est également possible d'observer les séquelles d'un infarctus à partir de la contractilité, c'est-à-dire la valeur maximale de la contrainte active. Cette mesure de contractilité peut être réalisée sur un cycle de systole entier. Une contractilité inférieure à la normale indique alors une zone impactée par un infarctus.

Annexe A

(1)

$$M.F - r.V^2\, F = 0$$

(2)

$$[\text{Math. 1}]$$

$$\begin{pmatrix} s_{1D} * n_1{}^2 + C^{(2)} & 0 & 0 \\ 0 & s_{1D} * n_1{}^2 & 0 \\ 0 & 0 & s_{1D} * n_1{}^2 \end{pmatrix}$$

(3)

$$V = n_1.(\, s_{1D}/\, r\, )^{1/2}$$

(4)

$$s_{1D} = r.V^2$$

(5)

$$p = a_{cor}.e.s_{1D}$$

**Revendications**

**1.** Dispositif cardiaque, comprenant un émetteur (10) agencé pour émettre au moins une onde, une sonde (12) agencée pour mesurer une onde de cisaillement suscitée par une onde issue de l'émetteur (10), un détecteur (14) agencé pour détecter une phase de systole dans un signal électrocardiographique, et un estimateur (16) agencé pour déterminer, pendant au moins un cycle cardiaque, la vitesse de propagation d'une pluralité d'ondes de cisaillement suscitées par l'émission d'ondes selon plusieurs directions vers le coeur d'un patient, pour déterminer à l'aide du détecteur (14) celle qui présente durant la phase de systole une vitesse de propagation maximale, et pour en tirer une contrainte active cardiaque (160).

**2.** Dispositif cardiaque selon la revendication 1, comprenant un calculateur (22) agencé pour déterminer une pression ventriculaire (220) à partir d'une épaisseur de paroi ventriculaire, d'un rayon de cavité ventriculaire et de la contrainte active (160) déterminée par l'estimateur (16).

**3.** Dispositif cardiaque selon la revendication 2, comprenant un analyseur d'image (20) agencé pour déterminer l'épaisseur de paroi ventriculaire et le rayon de cavité ventriculaire sur la base d'au moins une image cardiaque.

**4.** Dispositif cardiaque selon la revendication 3, dans lequel l'analyseur d'image (20) est agencé pour déterminer l'épaisseur de paroi ventriculaire et le rayon de cavité ventriculaire sur la base d'au moins une image cardiaque tirée d'une imagerie par ondes de cisaillement.

**5.** Dispositif cardiaque selon la revendication 4, comprenant en outre un imageur (24) agencé pour fournir l'imagerie par ondes de cisaillement à partir d'au moins une onde de cisaillement mesurée par la sonde (12).

**6.** Dispositif cardiaque selon l'une des revendications précédentes, dans lequel l'au moins une onde issue de l'émetteur (10) est un ultrason de compression.

**7.** Dispositif cardiaque selon l'une des revendications précédentes, dans lequel l'émetteur (10) et la sonde (12) sont mis en oeuvre par une sonde d'échocardiographie bidimensionnelle qui présente une direction de sonde définissant une direction de propagation autour de laquelle elle est pivotable, et dans lequel l'estimateur (16) est agencé pour déterminer la vitesse de propagation maximale sur la base de mesures réalisées par la sonde (12) pour trois directions coplanaires et non colinéaires.

**8.** Dispositif cardiaque selon l'une des revendications 1 à 6, dans lequel l'émetteur (10) et la sonde (12) sont mis en oeuvre par une sonde ultrason tridimensionnelle.

**9.** Dispositif cardiaque selon la revendication 8, dans lequel la sonde ultrason tridimensionnelle est une sonde d'échocardiographie tridimensionnelle.

**Patentansprüche**

**1.** Kardiales Gerät, das einen Emitter (10), der eingerichtet ist, mindestens eine Welle zu emittieren, eine Sonde (12), die eingerichtet ist, eine von einer vom Emitter (10) stammenden Welle hervorgerufene Scherwelle zu messen, einen Detektor (14), der eingerichtet ist, eine Systolenphase in einem elektrokardiographischen Signal zu erkennen, und eine Schätzfunktion (16) enthält, die eingerichtet ist, um während mindestens eines Herzzyklus die Ausbreitungsgeschwindigkeit einer Vielzahl von Scherwellen zu bestimmen, die durch die Emission von Wellen gemäß mehreren Richtungen zum Herzen eines Patienten hervorgerufen werden, um mit Hilfe des Detektors (14) diejenige zu bestimmen, die während der Systolenphase eine maximale Ausbreitungsgeschwindigkeit aufweist, und um daraus eine aktive Herzbeanspruchung (160) abzuleiten.

**2.** Kardiales Gerät nach Anspruch 1, das einen Rechner (22) enthält, der eingerichtet ist, um einen Ventrikeldruck (220) ausgehend von einer Ventrikelwanddicke, einem Ventrikelhohlraumradius und der von der Schätzfunktion (16) bestimmten aktiven Beanspruchung (160) zu bestimmen.

**3.** Kardiales Gerät nach Anspruch 2, das einen Bildanalysator (20) enthält, der eingerichtet ist, um die Ventrikelwanddicke und den Ventrikelhohlraumradius auf der Basis mindestens eines kardialen Bilds zu bestimmen.

**4.** Kardiales Gerät nach Anspruch 3, wobei der Bildanalysator (20) eingerichtet ist, um die Ventrikelwanddicke und den Ventrikelhohlraumradius auf der Basis mindestens eines aus einer Bildgebung durch Scherwellen entnommenen kardialen Bilds zu bestimmen.

5. Kardiales Gerät nach Anspruch 4, dass außerdem einen Bildgeber (24) enthält, der eingerichtet ist, um die Bildgebung durch Scherwellen ausgehend von mindestens einer von der Sonde (12) gemessenen Scherwelle zu liefern.

6. Kardiales Gerät nach einem der vorhergehenden Ansprüche, wobei die mindestens eine vom Emitter (10) stammende Welle ein Kompressionsultraschall ist.

7. Kardiales Gerät nach einem der vorhergehenden Ansprüche, wobei der Emitter (10) und die Sonde (12) von einer zweidimensionalen Echokardiographiesonde angewendet werden, die eine Sondenrichtung hat, die eine Ausbreitungsrichtung definiert, um die herum sie schwenkbar ist, und wobei die Schätzfunktion (16) eingerichtet ist, um die maximale Ausbreitungsrichtung auf der Basis von Messungen zu bestimmen, die von der Sonde (12) für drei koplanare und nicht kolineare Richtungen durchgeführt werden.

8. Kardiales Gerät nach einem der Ansprüche 1 bis 6, wobei der Emitter (10) und die Sonde (12) von einer dreidimensionalen Ultraschallsonde angewendet werden.

9. Kardiales Gerät nach Anspruch 8, wobei die dreidimensionale Ultraschallsonde eine dreidimensionale Echokardiographiesonde ist.

**Claims**

1. Cardiac device comprising a transmitter (10) arranged to transmit at least one wave, a probe (12) arranged to measure a shear wave caused by a wave coming from the transmitter (10), a detector (14) arranged to detect a systole phase in an electrocardiographic signal, and an estimator (16) arranged to determine, during at least one cardiac cycle, the propagation velocity of a plurality of shear waves caused by the transmission of waves in several directions towards the heart of a patient, to determine, using the detector (14), that which has a maximum propagation velocity during the systole phase, and to derive an active cardiac stress (160) therefrom.

2. Cardiac device according to claim 1, comprising a calculator (22) arranged to determine a ventricular pressure (220) from a ventricular wall thickness, a ventricular cavity radius and the active stress (160) determined by the estimator (16).

3. Cardiac device according to claim 2, comprising an image analyser (20) arranged to determine the ventricular wall thickness and the ventricular cavity radius on the basis of at least one cardiac image.

4. Cardiac device according to claim 3, wherein the image analyser (20) is arranged to determine the ventricular wall thickness and the ventricular cavity radius on the basis of at least one cardiac image derived from shear wave imaging.

5. Cardiac device according to claim 4, further comprising an imager (24) arranged to provide shear wave imaging from at least one shear wave measured by the probe (12).

6. Cardiac device according to one of the preceding claims, wherein the at least one wave coming from the transmitter (10) is compressional ultrasound.

7. Cardiac device according to one of the preceding claims, wherein the transmitter (10) and the probe (12) are implemented by a two-dimensional echocardiography probe which has a probe direction defining a direction of propagation about which it can rotate, and wherein the estimator (16) is arranged to determine the maximum propagation velocity on the basis of measurements carried out by the probe (12) for three coplanar and non-collinear directions.

8. Cardiac device according to one of claims 1 to 6, wherein the transmitter (10) and the probe (12) are implemented by a three-dimensional ultrasound probe.

9. Cardiac device according to claim 8, wherein the three-dimensional ultrasound probe is a three-dimensional echocardiography probe.

[Fig. 1]

[Fig. 2]

[Fig. 3]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016156989 A

- FR 2015050058 W

**Littérature non-brevet citée dans la description**

- **O. VILLEMAIN.** Myocardial Stiffness Evaluation Using Noninvasive Shear Wave Imaging in Healthy and Cardiomyopathie Adults. *JACC: Cardiovascular Imaging,* vol. 12 (7